# EUROPÄISCHE PATENTSCHRIFT

(11) **EP 2 614 820 B1**
(45) Veröffentlichungstag und Bekanntmachung des Hinweises auf die Patenterteilung: **12.06.2019**
(21) Anmeldenummer: 13000059.9
(22) Anmeldetag: 08.01.2013
(51) Int. Cl.: A61K 9/70, A61K 31/27, A61K 47/32

(54) **Transdermales therapeutisches System mit Cholinesterase-Hemmer**
TRANSDERMAL THERAPEUTIC SYSTEM WITH A CHOLINE ESTERASE INHIBITOR
SYSTÈME THÉRAPEUTIQUE TRANSDERMIQUE DOTÉ D'UN INHIBITEUR DE LA CHOLINESTÉRASE

(30) Priorität: 11.01.2012 DE 102012000369
(43) Veröffentlichungstag der Anmeldung: 17.07.2013
(73) Patentinhaber: AMW GmbH, 83627 Warngau (DE)
(72) Erfinder: Lang, Susanne, 83627 Warngau (DE); Sahr, Florian, 83627 Warngau (DE); Pfaller, Tobias, 83627 Warngau (DE); Wolff, Katharina, 22767 Hamburg (DE); Fitzner, Ansgar, 22767 Hamburg (DE)
(74) Vertreter: Beckord & Niedlich Patentanwälte PartG mbB

(56) Entgegenhaltungen:
- EP-A1- 2 172 194
- WO-A1-2007/064407
- WO-A1-2012/007150
- WO-A1-2012/080365

## Beschreibung

Die Erfindung betrifft ein transdermales therapeutisches System (TDS) mit einem Gehalt an einem Cholinesterase-Hemmer vom Carbamat-Typ als Wirkstoff, beispielsweise mit Rivastigmin.

Um Rivastigmin über ein transdermales System (TDS) zu verabreichen, ist nach dem Stand der Technik ein Antioxidans nötig, da sonst der Wirkstoff chemisch nicht stabil bleibt. EP 1 047 409 B1**,** WO 99/34 782 und das Exelon® Patch sehen beispielsweise alpha-Tocopherol vor. Für das Antioxidans alpha-Tocopherol ist in der Literatur beschrieben, dass es allergische Kontakt-Dermatitis verursachen kann. Verwiesen sei beispielsweise auf Dermatitis 2010 June 21(3):148-53 (Kosari, P., Alikhan, A., Sockolov, M. & Feldman, SR.: Vitamin E and allergic contact dermatitis), Dermatitis 2010 August 21(4):199-202 (Adams, AK. & Connolly, SM.: Allergic contact dermatitis from vitamin E: the experience at Mayo Clinic Arizona, 1987 to 2007) und Dermatitis 2008 May-June 19(3):154-6 (Ramírez Santos, A., Fernändez-Redondo, V., Pérez Pérez, L., Concheiro Cao, J. & Toribio, J.: Contact allergy from vitamins in cosmetic products).

Außerdem ist nach dem Stand der Technik eine Silikon-Klebeschicht erforderlich, um eine genügend starke Haftung auf der Haut zu erreichen. So wird in WO2007/064 407 A1 beschrieben, dass die Reservoirschicht (wie bei Exelon) zu geringe Klebkraft hat und es deswegen nötig ist, eine Silikon-Klebeschicht zu laminieren.

Ferner beschreiben DE 38 05 744 C2 und DE 38 44 992 B4 die Verwendung des S-(-)-Enantiomeren von Rivastigmin und eines seiner Salze für systemische transdermale Verabreichung zur Behandlung von Dementia und Alzheimer-Krankheit.

EP 1171 104 B1 und WO 00/64 418 beschreiben eine transdermale therapeutische Matrix bzw. ein Reservoir-System mit einem basischen oder neutralen Wirkstoff, beispielsweise Rivastigmin, sowie ein vollständig oder partiell neutralisiertes saures druckempfindliches Polyacrylat-Klebemittel.

Des Weiteren betrifft EP 2 172 194 A1 ein transdermales Wirkstoffabgabesystem für flüssige Wirkstoffe, wobei sich die Wirkstoffe in einem Acrylatpolymer befinden.

EP 2 292 219 A1**,** EP 1 959 937 und EP 2 286 802 beschreiben ein transdermales therapeutisches System mit Rivastigmin für AUC24h von 25 bis 450 ng^{∗}h/mL bei wiederholter einmaliger Tagesdosis.

Ferner beschreibt WO 2011/076 621 A2 ein transdermales therapeutisches System mit Rivastigmin als Wirkstoff in einem Reservoir mit einer Polymermatrix, die weder Hydroxylnoch Carboxyl-Gruppen aufweist.

Es fällt auf, das der Stand der Technik identische Matrix-Materialien verwendet, und zwar DuroTak 387-2051 und 87-2353. Bei DuroTak 87-2353 handelt es sich nach US 2010/0 087 768 A1 Seite 5 [0085] um "a random copolymer of 2-ethylhexylacrylatre (2-EHA) 62.2 %, methylacrylate (MA) 32.0 %, glacial acrylic acid (GAA) 5.7 % and glycidylmethacrylate (GMA) 0.03 % in an organic solvent solution consisting of ethylacetate and hexane in the ratio 86.9:13.1, the copolymer is supplied at 35-38 % solid in solution."

| | | |
|---|---|---|
| DuroTak | 387-2051 | 87-2353 |
| WO 99/34 782 A1 | Seite 2 Abs. 3 mit Seite 7 Abs. 3 | Seite 2 Abs. 3 |
| WO 00/64 418 A2 | Seite 15 Abs. 2 | |
| US 2010/0 087 768 A1 | Seite 4 Tab. 1 | Seite 5 [0085] |
| WO 2007/064 407 A1 | | Seite 6 Abs. 4, Seite 12 Example |

Auch die Verwendung von Avocadoöl für dermale und transdermale Applikationen ist bereits bekannt.

So beschreibt DE 698 30 095 T2 (Priorität 11. 04. 1997) ein kosmetisches oder dermopharmazeutisches Pflaster zur Freisetzung eines in Form von Partikeln vorliegenden wasserlöslichen Wirkstoffs und zusätzlich eines in einem Öl gelösten fettlöslichen Wirkstoffs in der Epidermis [0001]. Als Öl wird in einer großen Liste von Ölen unter anderem auch Avocadoöl vorgeschlagen. Avocadoöl wird auch von DE 198 82 916 T1 (Priorität 28. 12. 1997) (Seite 7) gleichfalls in einer Liste von Ölen zur Hautpflege und zum Hautschutz vorgeschlagen, hier allerdings in einer Gelzusammensetzung. Auch die in DE 199 11 262 A1 (Anmeldetag 13. 03. 1999) beschriebene Vorrichtung zur Abgabe kosmetischer Wirkstoffe kann sich des Avocadoöls bedienen, das wiederum in einer großen Liste von Ölen angeführt wird. Zuletzt erwähnt DE 600 28 642 T2 (Priorität 31. 03. 1999) Avocadoöl in einer umfassenden Liste von Ölen zur Einarbeitung eines solubilisierten Wirkstoffs [0020], und zwar für ein Pflaster mit einem Gehalt an magnetischen Partikeln, die die Penetration des Wirkstoffes in die Haut fördern [0009], wobei mit dem Pflaster auf der Haut eine kosmetische Wirkung erzielt und/oder eine pharmazeutische Behandlung durchgeführt werden soll [0001].

Die Verwendung von Avocadoöl ist auch für TDSe erwähnt worden, wurde jedoch seit längerem für TDSe nicht mehr aufgegriffen. So beschrieben DE 33 47 278 A1 (Anmeldetag 28. 12. 1983) und DE 34 09 079 A1 (Anmeldetag 13. 03. 1984) antiphlogistische medizinische Pflaster, für die jeweils in einer Liste von Ölen als Schleppmittel unter anderem Avocadoöl angeführt wurde (Seite 12 bzw. Seite 9). Ferner beschrieb DE 42 37 453 C1 (Anmeldetag 06. 11. 1992) ein TDS zur Abgabe eines speziellen Wirkstoffs, nämlich 17-ß-Estradiol, wobei Avocadoöl in einer umfangreichen Liste möglicher die Löslichkeit verbessernder Zusätze bzw. Hautpermeationsverbesserer angeführt wird (Seite 2, Zeile 64 bis Seite 3, Zeile 13).

Aufgabe der Erfindung ist es, ein transdermales therapeutisches System zur Behandlung von leichter bis mittelschwerer Demenz vom Typ der Alzheimer-Krankheit und mit einem Gehalt an einem Cholinesterase-Hemmer vom Carbamat-Typ wie Rivastigmin für die Behandlung von leichter bis mittelschwerer Demenz vom Typ der Alzheimer-Krankheit vorzusehen.

Mit dem transdermalen therapeutischen System sollen (1) die mit oraler Gabe einhergehenden häufigen bis sehr häufigen Nebenwirkungen umgangen werden, insbesondere Übelkeit, Durchfall und Erbrechen. (2) Auch sollte das transdermale therapeutische System eine bessere Compliance als orale Arzneiformen aufweisen. (3) So sollte das transdermale therapeutische System eine bequeme Applikation auf die Haut erlauben und (4) eine gute Hautverträglichkeit bei gutem Tragekomfort und ausreichender Klebkraft bieten. Außerdem sollte es (5) eine hohe Lagerstabilität und (6) eine zuverlässige Wirksamkeit bieten. (7) Das transdermale therapeutische System sollte kostengünstig herzustellen sein. (8) Es sollte für eine lang anhaltende kontinuierliche Wirkstofffreigabe von insbesondere 1 bis 7 Tagen einsetzbar sein. (9) Schließlich sollte das transdermale therapeutische System auch ohne Antioxidans vorgesehen werden können.

Gegenstand der Erfindung ist nun ein transdermales therapeutisches System frei von Antioxidans und
- mit einer wirkstoffundurchlässigen Abdeckschicht,
- mit einem Wirkstoffreservoir mit einem Gehalt an
   - mindestens einem Cholinesterase-Hemmer vom Carbamat-Typ als Wirkstoff sowie
   - einem fakultativ druckempfindlichen Polymer umfassend oder bestehend aus einem Polyacrylat,
- mit einer applikationsseitigen wirkstofffreien Haftschicht, sofern das Polymer des Wirkstoff-Reservoirs nicht druckempfindlich ist, oder
- einer applikationsseitigen Haftschicht mit einem Gehalt an mindestens einem Cholinesterase-Hemmer vom Carbamat-Typ als Wirkstoff, wobei diese Schicht aus einem Polyacrylat mit freien OH Gruppen als Haftmittel besteht, und
- mit einer Abziehschicht.

Das erfindungegemäße transdermale System kann also auch ohne gesonderte Haftschicht vorgesehen werden. Dazu kann das Polymer des Wirkstoff-Reservoirs druckempfindlich und damit haftend vorgesehen sein.

Unter dem Begriff Polymer kann auch ein Polymergemisch verstanden werden. Unter einer wirkstofffreien Haftschicht ist eine Schicht zu verstehen, die bei der Herstellung nicht mit Wirkstoff beladen worden ist.

Erfindungsgemäß kann für das transdermale therapeutische System Rivastigmin als Cholinesterase-Hemmer vom Carbamat-Typ vorgesehen sein. Der Wirkstoff kann auch als physiologisch verträgliches Salz, Hydrat, Solvat oder Derivat vorgesehen werden.

Erfindungsgemäß kann ferner für das transdermale therapeutische System Rivastigmin und/oder mindestens ein anderer Cholinesterase-Hemmer vom Carbamat-Typ vorgesehen sein. Das erfindungsgemäße transdermale therapeutische System kann ein oder mehrere Cholinesterase-Hemmer vom Carbamat-Typ umfassen, wobei Rivastigmin dieser Wirkstoff oder einer dieser Wirkstoffe sein kann.

Erfindungsgemäß kann für das transdermale therapeutische System eine Wirkstoff-Konzentration im Wirkstoffreservoir von 0,1 bis 40 und insbesondere bis 50 Gew.-% (bezogen auf das Gesamtgewicht des Wirkstoffreservoirs) vorgesehen sein.

Ferner kann erfindungsgemäß für das transdermale therapeutische System eine Wirkstoff-Konzentration im Wirkstoffreservoir von 15 bis 35 Gew.-% und insbesondere etwa 30 Gew.-% (bezogen auf das Gesamtgewicht des Wirkstoffreservoirs) vorgesehen sein.

Bei dem erfindungsgemäßen transdermalen therapeutischen System kann der mindestens eine Cholinesterase-Hemmer vom Carbamat-Typ im Wirkstoffreservoir gelöst oder homogen dispergiert vorliegen.

Für das erfindungsgemäße transdermale therapeutische System können von 0 bis zu 15 Gew.-% Avocadoöl und/oder Palmöl (bezogen auf das Gesamtgewicht des Wirkstoffreservoirs) vorgesehen sein.

Auch kann das erfindungsgemäße transdermale therapeutische System einen Gehalt an Avocadoöl und/oder Palmöl und einen zusätzlichen Gehalt an mindestens einem weiteren fakultativen Hilfsstoff aufweisen, der beispielsweise zur Kohäsionsverbesserung oder zur Löslichkeitsverbesserung beiträgt, insbesondere zu verbesserter Löslichkeit des Wirkstoffs.

Bei dem erfindungsgemäßen transdermalen therapeutischen System können das Avocadoöl und/oder Palmöl und der fakultative zusätzliche Hilfsstoff im Wirkstoffreservoir gelöst oder homogen dispergiert vorliegen.

Bei dem erfindungsgemäßen transdermalen therapeutischen System kann das nicht-druckempfindliche oder fakultativ druckempfindliche Polymer des Wirkstoff-Reservoirs Polyacrylate mit freien Carboxyl-Gruppen, insbesondere DuroTak 87-235A bzw. 235A, und/oder Polyacrylate mit freien OH-Gruppen, insbesondere DuroTak 387-2510, 87-2510 bzw. 2510, und/oder Polyacrylate mit quaternären Ammonium-Gruppen, oder eine fakultativ druckempfindliche Mischung mehrerer dieser Polymere umfassen oder daraus bestehen.

So kann für das erfindungsgemäße transdermale therapeutische System das Wirkstoff-Reservoir als Polyacrylat mit quaternären Ammonium-Gruppen ein Copolymer aus Ethylacrylat, Methylmethacrylat und Methacrylsäureester mit quaternären Ammonium-Gruppen, vorzugsweise Trimethylammonioethyl-methacrylat-chlorid, umfassen oder daraus bestehen.

So kann es sich bei dem erfindungsgemäßen transdermalen therapeutischen System um Eudragit RL 100, Eudragit RS 100, Eudragit RL PO und/oder Eudragit RS PO als Polyacrylat mit quaternären Ammonium-Gruppen handeln.

Für Eudragit wird hier der Offenbarungsgehalt folgender Dokumente miteinbezogen:
DE 39 39 376, DE 40 20 144 und DE 43 10 012;
EP 0 617 972, EP 0 848 960 und EP 1 061 900;
WO 99/34 782 A1 Seite 3 und WO 06/12 966.

Bei dem erfindungsgemäßen transdermalen therapeutischen System können sich im Wirkstoff-Reservoir die Anteile
- Polyacrylat mit freien Carboxyl-Gruppen zu
- Polyacrylat mit freien OH-Gruppen zu
- Polyacrylat mit quaternären Ammonium-Gruppen verhalten wie 3 bis 4 zu 4 bis 1 zu 0 bis 2 und zu 100 % ergänzen (Gewichtsbasis).

Ferner kann bei dem erfindungsgemäßen transdermalen therapeutischen System das Polacrylat oder Polyacrylat-Gemisch des Wirkstoff-Reservoirs mit einem Klebemittel (tackifier) auf natürlicher oder synthetischer Kohlenwasserstoff-basis modifiziert sein.

Ferner kann bei dem erfindungsgemäßen transdermalen therapeutisches System das Wirkstoff-Reservoir mit einer applikationsseitigen Haftschicht mit einem Gehalt an Cholinesterase-Hemmer vom Carbamat-Typ als Wirkstoff versehen sein, vorzugsweise mit einem Gehalt an Rivastigmin, oder mit einer vorstehend angesprochenen WirkstoffMischung, wobei diese Haftschicht oder Schicht als Haft oder Haftmittel insbesondere DuroTak 387-2510, 87-2510 bzw. 2510 umfassen bzw. daraus bestehen kann.

Dabei kann die Wirkstoff-Konzentration in der applikationsseitigen Haftschicht 0,1 bis 40 und insbesondere bis 50 Gew.-%, vorzugsweise 15 bis 35 Gew.-% und insbesondere etwa 30 Gew.-% (bezogen auf das Gesamtgewicht der Haftschicht) betragen.

Bei dem erfindungsgemäßen transdermalen therapeutischen System kann der mindestens eine Cholinesterase-Hemmer vom Carbamat-Typ in der Haftschicht gelöst oder homogen dispergiert vorliegen.

Bei dem erfindungsgemäßen transdermalen therapeutischen System können sich
-- im Wirkstoff-Reservoir die Anteile
   - Polyacrylat mit freien Carboxyl-Gruppen zu
   - Polyacrylat mit quaternären Ammonium-Gruppen verhalten wie 4 bis 6 zu 3 bis 1 und zu 100 % ergänzen (Gewichtsbasis) und
-- in der applikationsseitigen Haftschicht der Anteil an
   - Polyacrylat mit freien Hydroxyl-Gruppen 100 % betragen (Gewichtsbasis).

Bei dem erfindungsgemäßen transdermalen therapeutischen System sind das Wirkstoff-Reservoir und fakultative Haftschichten und insbesondere das Wirkstoff-Reservoir frei von Antioxidanz. Das erfindungsgemäße transdermale therapeutische System weist also keinen Gehalt an einem Antioxidanz auf, ist also frei davon, beispielsweise wenn das Wirkstoff-Reservoir als nicht-druckempfindliches oder fakultativ druckempfindliches Polymer ein Carboxylgruppen aufweisendes unvernetztes Polyacrylat wie DuroTak DT 87-235 A umfasst oder daraus besteht.

Ferner kann das erfindungsgemäße transdermale therapeutische System einen Gehalt an einem Lösungsvermittler, insbesondere einen Gehalt an 1,2-Propandiol, Dimethylacetamid und/oder N-Methylpyrrolidon aufweisen.

Ferner kann das erfindungsgemäße transdermale therapeutische System einen Gehalt an Kohäsionsförderer, insbesondere einen Gehalt an Polysiloxan, aufweisen.

Ferner kann das erfindungsgemäße transdermale therapeutische System einen Gehalt an Klebkraftförderer, insbesondere einen Gehalt an einem oder mehreren Harzen natürlichen oder synthetischen Ursprungs aufweisen.

Ferner kann das erfindungsgemäße transdermale therapeutische System einen Gehalt an einem Permeationsförderer aufweisen.

Ferner kann das erfindungsgemäße transdermale therapeutische System auf eine Wirkstoff-Hautpermeation in vitro von 10 µg (Mikrogramm)/(h und System) bis 5 mg/(h und System) eingestellt sein.

Ferner kann bei dem erfindungsgemäßen transdermalen therapeutischen System der Gehalt an Wirkstoff auf eine kontinuierliche Wirkstoff-Freigabe für eine Dauer von ganzen Tagen des Bereichs von 1 bis 7 Tagen eingestellt sein.

Schließlich kann das erfindungsgemäße transdermale therapeutische System mit
- einem Flächengewicht des Wirkstoff-Reservoirs von 30 bis 150, vorzugsweise 35 bis 60, insbesondere 35 bis 45 und bevorzugt etwa 40 g/m² und
- einem Flächengewicht der applikationsseitigen Haftschicht mit einem Gehalt an Cholinesterase-Hemmer vom Carbamat-Typ als Wirkstoff von 10 bis 30 und insbesondere etwa 20 g/m² vorgesehen sein.

Die Abziehschicht kann eine wirkstoffundurchlässige Schutzschicht sein, beispielsweise eine silikonisierte Polyesterfolie, die vor dem Aufbringen auf die Haut abgezogen wird Nachstehend wird die Erfindung durch Beispiele näher erläutert.

### Beispiel 1

Es wurde Rivastigmin in Ethylacetat verdünnt. Zu der erhaltenen Lösung wurde ein Polyacrylat-Gemisch hinzugefügt, wonach homogenisiert wurde. Der folgenden Tabelle sind Mengenangaben (Trockengewicht) zu entnehmen. Die erhaltenen Mischung wurde auf eine Polyesterfolie bis zu einem Gewicht von 60 g Homogenisat/m² aufgestrichen. Danach wurde das Lösungsmittel entfernt, indem die Folie mit aufgestrichenem Homogenisat 15 min bei 60 °C getrocknet wurde. Auf den getrockneten Klebstoff-Film wurde eine silikonisierte Polyesterfolie kaschiert.

| Bestandteil | [Gew.-%] |
|---|---|
| | |
| Rivastigmin | 30 |
| Polyacrylat mit freien Carboxyl-Gruppen DuroTak 235 | 30 |
| Polyacrylat mit freien OH-Gruppen DuroTak2510 | 40 |
| Polyacrylat mit quaternären Ammonium-Gruppen | 0 |

### Beispiel 2

Er wurde Beispiel 1 mit folgender Rezeptur wiederholt.

| Bestandteil | [Gew.-%] |
|---|---|
| | |
| Rivastigmin | 30 |
| Polyacrylat mit freien Carboxyl-Gruppen DuroTak 235 | 40 |
| Polyacrylat mit freien OH-Gruppen DuroTak2510 | 10 |
| Polyacrylat mit quaternären Ammonium-Gruppen Eudragit RS | 20 |

### Beispiel 3

Er wurde Beispiel 1 mit folgender Rezeptur wiederholt.

| Bestandteil | [Gew.-%] |
|---|---|
| | |
| Rivastigmin | 30 |
| Polyacrylat mit freien Carboxyl-Gruppen DuroTak 235 | 40 |
| Polyacrylat mit freien OH-Gruppen DuroTak2510 | 20 |
| Polyacrylat mit quaternären Ammonium-Gruppen Eudragit RS | 10 |

### Beispiel 4

Er wurde Beispiel 1 mit folgender Rezeptur für das Wirkstoff-Reservoir wiederholt, wobei die Polyacrylat-Mischung auf die Polyesterfolie bis zu einem Gewicht von 40 g Homogenisat/m² aufgestrichen wurde.

| Bestandteil | [Gew.-%] |
|---|---|
| | |
| Rivastigmin | 30 |
| Polyacrylat mit freien Carboxyl-Gruppen DuroTak 235A | 50 |
| Polyacrylat mit freien OH-Gruppen DuroTak2510 | 0 |
| Polyacrylat mit quaternären Ammonium-Gruppen Eudragit RL | 20 |

Auf das fertige Wirkstoff-Reservoir wurde eine Haftschicht mit folgenden Bestandteilen bis zu einem Gewicht von 20 g Homogenisat/m² aufgetragen:

| Bestandteil | [Gew.-%] |
|---|---|
| | |
| Rivastigmin | 30 |
| Polyacrylat mit freien Carboxyl-Gruppen | 0 |
| Polyacrylat mit freien OH-Gruppen DuroTak2510 | 70 |
| Polyacrylat mit quaternären Ammonium-Gruppen Eudragit | 0 |

### Beispiel 5

Er wurde Beispiel 4 mit folgender Rezeptur für das Wirkstoff-Reservoir wiederholt, wobei die Polyacrylat-Mischung auf die Polyesterfolie bis zu einem Gewicht von 40 g Homogenisat/m² aufgestrichen wurde.

| Bestandteil | [Gew.-%] |
|---|---|
| | |
| Rivastigmin | 30 |
| Polyacrylat mit freien Carboxyl-Gruppen DuroTak 235A | 50 |
| Polyacrylat mit freien OH-Gruppen | 0 |
| Polyacrylat mit quaternären Ammonium-Gruppen Eudragit RS | 20 |

Auf das fertige Wirkstoff-Reservoir wurde eine Haftschicht mit den Bestandteilen gemäß Beispiel 4 bis zu einem Gewicht von 20 g Homogenisat/m² aufgetragen.

## Patentansprüche

1. Transdermales therapeutisches System frei von Antioxidanz und
- mit einer wirkstoffundurchlässigen Abdeckschicht,
- mit einem Wirkstoffreservoir mit einem Gehalt an
- mindestens einem Cholinesterase-Hemmer vom Carbamat-Typ als Wirkstoff sowie
- einem fakultativ druckempfindlichen Polymer umfassend oder bestehend aus einem Polyacrylat,
- einer applikationsseitigen Haftschicht mit einem Gehalt an Cholinesterase-Hemmer vom Carbamat-Typ als Wirkstoff, wobei diese Schicht aus einem Polyacrylat mit freien OH-Gruppen als Haftmittel besteht, und
- mit einer Abziehschicht.

2. Transdermales therapeutisches System nach Anspruch 1 mit Rivastigmin als Cholinesterase-Hemmer und/oder mindestens einem anderen Cholinesterase-Hemmer vom Carbamat-Typ.

3. Transdermales therapeutisches System nach Anspruch 1 oder 2 mit einer Wirkstoff-Konzentration im Wirkstoffreservoir von 0,1 bis 40 und insbesondere bis 50 Gew.-%, vorzugsweise 15 bis 35 Gew.-% und insbesondere von etwa 30 Gew.-% (bezogen auf das Gesamtgewicht des Wirkstoffreservoirs).

4. Transdermales therapeutisches System nach mindestens einem der vorhergehenden Ansprüche, bei dem der mindestens eine Cholinesterase-Hemmer vom Carbamat-Typ im Wirkstoffreservoir gelöst oder homogen dispergiert vorliegt.

5. Transdermales therapeutisches System nach mindestens einem der vorhergehenden Ansprüche, bei dem das nicht-druckempfindliche oder fakultativ druckempfindliche Polymer des Wirkstoff-Reservoirs Polyacrylat mit freien Carboxyl-Gruppen, und/oder Polycrylat mit freien OH-Gruppen, und/oder Polyacrylat mit quaternären Ammonium-Gruppen, oder eine fakultativ druckempfindliche Mischung mehrerer dieser Polymere umfasst oder daraus besteht.

6. Transdermales therapeutisches System nach Anspruch 5, bei dem das Polymer des Wirkstoff-Reservoirs als Polyacrylat mit quaternären Ammonium-Gruppen ein Copolymer aus Ethylacrylat, Methylmethacrylat und Methacrylsäureester mit quaternären Ammonium-Gruppen, vorzugsweise Trimethylammonioethyl-methacrylat-chlorid, umfasst oder daraus besteht.

7. Transdermales therapeutisches System nach Anspruch 5 und/oder 6 mit Polyacrylat mit quaternären Ammonium-Gruppen.

8. Transdermales therapeutisches System nach Anspruch 5 und/oder 6 und/oder 7, bei dem sich im Wirkstoff-Reservoir die Anteile
- Polyacrylat mit freien Carboxyl-Gruppen zu
- Polyacrylat mit freien OH-Gruppen zu
- Polyacrylat mit quaternären Ammonium-Gruppen verhalten wie 3 bis 4 zu 4 bis 1 zu 0 bis 2 und zu 100 % ergänzen (Gewichtsbasis).

9. Transdermales therapeutisches System nach mindestens einem der vorhergehenden Ansprüche, bei dem das Wirkstoff-Reservoir mit einer applikationsseitigen Haftschicht mit einem Gehalt an Cholinesterase-Hemmer vom Carbamat-Typ als Wirkstoff versehen ist, wobei diese Haftschicht oder Schicht als Haft oder Haftmittel ein Polyacrylat mit freien OH-Gruppen umfasst bzw. daraus besteht.

10. Transdermales therapeutisches System nach Anspruch 9 mit einem Wirkstoff gemäß Anspruch 2 und vorzugsweise mit einer Wirkstoff-Konzentration gemäß Anspruch 3, und zwar von 0,1 bis 40 und insbesondere bis 50 Gew.-%, vorzugsweise 15 bis 35 Gew.-% und insbesondere von etwa 30 Gew.-% (bezogen auf das Gesamtgewicht der Haftschicht).

11. Transdermales therapeutisches System nach Anspruch 9 oder 10, bei dem sich
-- im Wirkstoff-Reservoir die Anteile
- Polyacrylat mit freien Carboxyl-Gruppen zu
- Polyacrylat mit quaternären Ammonium-Gruppen verhalten wie 4 bis 6 zu 3 bis 1 und zu 100 % ergänzen (Gewichtsbasis) und
-- in der applikationsseitigen Haftschicht der Anteil an
- Polyacrylat mit freien Hydroxyl-Gruppen 100 % beträgt (Gewichtsbasis).

12. Transdermales therapeutisches System nach mindestens einem der vorhergehenden Ansprüche, **dadurch gekennzeichnet, dass** das System auf eine Wirkstoff-Hautpermeation in vitro von 10 µg (Mikrogramm)/(h und System) bis 5 mg/(h und System) eingestellt ist.

13. Transdermales therapeutisches System nach mindestens einem der vorhergehenden Ansprüche, **dadurch gekennzeichnet, dass** der Gehalt an Wirkstoff auf eine kontinuierliche Wirkstoff-Freigabe für eine Dauer von ganzen Tagen des Bereichs von 1 bis 7 Tagen eingestellt ist.

14. Transdermales therapeutisches System nach mindestens einem der vorhergehenden Ansprüche mit
- einem Flächengewicht des Wirkstoff-Reservoirs von 30 bis 150, vorzugsweise 35 bis 60, insbesondere 35 bis 45 und bevorzugt etwa 40 g/m² und
- einem Flächengewicht der applikationsseitigen Haftschicht mit einem Gehalt an Cholinesterase-Hemmer vom Carbamat-Typ als Wirkstoff von 10 bis 30 und insbesondere etwa 20 g/m².

## Claims

1. Transdermal therapeutic system free of antioxidants and
- with an active substance-impermeable cover layer,
- with an active substance reservoir with a content of
- at least one carbamate-type cholinesterase inhibitor as the active substance as well as
- an optional pressure-sensitive polymer comprising or consisting of a polyacrylate
- an application-side adhesive layer with a content of carbamate-type cholinesterase inhibitor as active substance, wherein this layer consists of a polyacrylate with free OH groups as adhesive agent, and
- with a peel-off layer.

2. Transdermal therapeutic system according to claim 1 with Rivastigmine as the cholinesterase inhibitor and/or at least one other carbamate-type cholinesterase inhibitor.

3. Transdermal therapeutic system according to claim 1 or 2 with an active substance concentration in the active substance reservoir of 0.1 to 40 and in particular up to 50% by weight, preferably 15 to 35% by weight and especially of around 30% by weight (in relation to the total weight of the active substance reservoir).

4. Transdermal therapeutic system according to at least one of the preceding claims in which at least one carbamate-type cholinesterase inhibitor is present in dissolved or homogenously dispersed form in the active substance reservoir.

5. Transdermal therapeutic system according to at least one of the preceding claims in which the non-pressure-sensitive or optionally pressure-sensitive polymer of the active substance reservoir comprises or consists of polyacrylate with free carboxyl groups, and/or polyacrylate with free OH groups, and/or polyacrylate with quaternary ammonium groups, or an optionally pressure-sensitive mixture of several of these polymers.

6. Transdermal therapeutic system according to claim 5 in which the polymer of the active substance reservoir as polyacrylate with quaternary ammonium groups comprises or consists of a copolymer of ethyl acrylate, methyl methacrylate, methacrylic acid ester with quaternary ammonium groups, preferably trimethylammonio-ethyl methacrylic chloride.

7. Transdermal therapeutic system according to claim 5 and/or 6 with polyacrylate with quaternary ammonium groups.

8. Transdermal therapeutic system according to claim 5 and/or 6 and/or 7 in which in the active substance reservoir the proportions
- polyacrylate with free carboxyl groups to
- polyacrylate with free OH groups to
- polyacrylate with quaternary ammonium groups are 3 to 4 to 4 to 1 to 0 to 2 and complement each other 100% (based on weight).

9. Transdermal therapeutic system according at least one of the preceding claims in which the active substances reservoir is provided with an application-side adhesive layer with a content of carbamate-type cholinesterase inhibitor as active substance, wherein this adhesive layer or layer comprises or consists of a polyacrylate with free OH groups as adhesive or adhesive agent.

10. Transdermal therapeutic system according to claim 9 with an active substance according to claim 2 and preferably with an active substance concentration according to claim 3, namely of 0.1 to 40 and in particular up to 50% by weight, preferably 15 to 35% by weight and especially of around 30% by weight (in relation to the total weight of the adhesive layer).

11. Transdermal therapeutic system according to claim 9 or 10 in which
- in the active substance reservoir the proportions
- polyacrylate with free carboxyl groups to
- polyacrylate with quaternary ammonium groups are 4 to 6 to 3 to 1 and complement each other 100% (based on weight and
- in the application-side adhesive layer the proportion of
- polyacrylate with free carboxyl groups is 100% (based on weight).

12. Transdermal therapeutic system according to at least one of the preceding claims **characterised in that** the system is adjusted to an active substance dermal permeation in vitro of 10 µg (micrograms)/(h and system) to 5 mg/(h and system).

13. Transdermal therapeutic system according to at least one of the preceding claims **characterised in that** the content of active substance is adjusted to continuous active substance release for a duration of whole days in the range 1 to 7 days.

14. Transdermal therapeutic system according to any one of the preceding claims with
- a weight per unit area of the active substance reservoir of 30 to 150, preferably 35 to 60, more particularly 35 to 45 and preferably around 40 g/m² and
- a weight per unit area of the application-side adhesive layer with a content of carbamate-type cholinesterase inhibitor as active substance from 10 to 30 and in particular around 20 g/m².

## Revendications

1. Système thérapeutique transdermique exempt d'antioxydant et
- pourvu d'une couche de recouvrement imperméable aux agents actifs,
- pourvu d'un réservoir d'agent actif, comprenant une teneur
- d'au moins un inhibiteur de la cholinestérase de type carbamate, en tant qu'agent actif, ainsi
- qu'un polymère facultativement sensible à la pression comprenant un polyacrylate ou constitué de celui-ci
- pourvu d'une couche adhérente du côté application, comprenant une teneur d'inhibiteur de la cholinestérase de type carbamate, en tant qu' agent actif, ladite couche étant constituée d'un polyacrylate à groupes OH libres, en tant qu'agent adhérent et
- pourvu d'une couche pelable.

2. Système thérapeutique transdermique selon la revendication 1 comportant de la rivastigmine en tant qu'inhibiteur de la cholinestérase et/ou au moins un autre inhibiteur de la cholinestérase de type carbamate.

3. Système thérapeutique transdermique selon la revendication 1 ou 2 comportant une concentration d'agent actif dans le réservoir d'gent actif de 0,1 à 40 et notamment à 50 % en poids, de préférence de 15 à 35 % en poids et notamment d'environ 30 % en poids (rapportés au poids total du réservoir d'agent actif).

4. Système thérapeutique transdermique selon au moins l'une quelconque des revendications précédentes, dans lequel au moins un inhibiteur de la cholinestérase de type carbamate est présent dans le réservoir d'agent actif sous forme dissoute ou en dispersion homogène.

5. Système thérapeutique transdermique selon au moins l'une quelconque des revendications précédentes, dans lequel le polymère insensible à la pression ou facultativement sensible à la pression du réservoir d' agent actif comprend un polyacrylate à groupes carboxyle libres et/ou un polyacrylate à groupes OH libres et/ou un polyacrylate à groupes ammonium quaternaires ou un mélange facultativement sensible à la pression de plusieurs desdits polymères ou en est constitué.

6. Système thérapeutique transdermique selon la revendication 5, dans lequel le polymère du réservoir d'agent actif comprend en tant que polyacrylate à groupes ammonium quaternaires un copolymère d'acrylate d'éthyle, de méthacrylate de méthyle et d'ester d'acide méthacrylique avec des groupes ammonium quaternaires, de préférence un chlorure de méthacrylate de triméthylammonioéthyle, ou en est constitué.

7. Système thérapeutique transdermique selon la revendication 5 et/ou 6 comportant un polyacrylate avec des groupes ammonium quaternaires.

8. Système thérapeutique transdermique selon la revendication 5 et/ou 6 et/ou 7, dans lequel, dans le réservoir d'agent actif, les parts
- de polyacrylate à groupes carboxyle libres au
- polyacrylate à groupes OH libres au
- polyacrylate à groupes ammonium quaternaires se présentent à raison de 3 - 4 à 4 - 1 à 0 -2 et se complètent à 100 % (base en poids).

9. Système thérapeutique transdermique selon au moins l'une quelconque des revendications précédentes, dans lequel le réservoir d'agent actif est muni d'une couche adhérente du côté application, comprenant une teneur d'inhibiteur de la cholinestérase de type carbamate, en tant qu'agent actif, ladite couche adhérente ou couche comportant en tant qu'adhérent ou agent adhérent un polyacrylate à groupes OH libres ou en étant constituée.

10. Système thérapeutique transdermique selon la revendication 9, comportant un agent actif selon la revendication 2 et de préférence dans une concentration d'agent actif selon la revendication 3, et à savoir de 0,1 à 40 et notamment à 50 % en poids, de préférence de 15 à 35 % en poids et notamment d'environ % en poids (en rapport au poids total de la couche adhérente).

11. Système thérapeutique transdermique selon la revendication 9 ou 10, dans lequel
- dans le réservoir d'agent actif, les parts
- du polyacrylate à groupes carboxyle libres
- au polyacrylate à groupes ammonium libres se présentent à raison de 4 - 6 à 3 - 1 et se complètent à 100 % (base en poids) et
- dans la couche adhérente du côté d'application, la part
- du polyacrylate à groupes hydroxyle libres s'élève à 100 % (base en poids).

12. Système thérapeutique transdermique selon au moins l'une quelconque des revendications précédentes, **caractérisé en ce que** le système est réglé à une perméation cutanée du agent actif in vitro de 10 µg (microgrammes)/(h et système) à 5 mg/(h et système).

13. Système thérapeutique transdermique selon au moins l'une quelconque des revendications précédentes, **caractérisé en ce que** la teneur en agent actif est réglée à une libération continue d'agent actif sur une durée de jours entiers de l'ordre de 1 à 7 jours.

14. Système thérapeutique transdermique selon au moins l'une quelconque des revendications précédentes comprenant
- un grammage du réservoir d'agent actif de 30 à 150, de préférence de 35 à 60, notamment de 35 à 45 et de manière préférentielle, d'environ 40 g/m² et
- un grammage de la couche adhérente du côté d'application avec une teneur d'inhibiteur de la cholinestérase de type carbamate en tant qu' actif de 10 à 30 et notamment d'environ 20 g/m².
